# EUROPEAN PATENT APPLICATION

(11) **EP 1 374 864 A1**
(43) Date of publication of application: **02.01.2004**
(21) Application number: 02018724.1
(22) Date of filing: 21.08.2002
(51) Int. Cl.: A61K 31/335, A61K 9/127, A61P 35/00

(54) **Amphiphilic taxane compositions**

(30) Priority: 26.06.2002 US 301245 P
(71) Applicant: Munich Biotech AG, 82061 Neuried (DE)
(72) Inventor: Fichert, Thomas, 82152 Krailling (DE); Schulze, Brita, 82432 Walchensee (DE); Uwe, Michaelis, 82362 Weilheim (DE); Teifel, Michael, 82377 Penzberg (DE); Haas, Heinrich, 81675 München (DE)
(74) Representative: Weiss, Wolfgang, Dipl.-Chem. Dr.

(57) **Abstract**

The present invention relates to novel compositions comprising a negatively charged derivative of a taxane drug and a positively charged amphiphile and formulations of this composition with a high drug to lipid ratio and high physicochemical stability for most efficient delivery of a pharmaceutically active amount of a taxane drug to a target site.

## Description

### Field of the invention

The present invention relates to novel compositions comprising a negatively charged derivative of a taxane drug and a positively charged amphiphile and formulations of this composition with a high drug to lipid ratio and high physicochemical stability for most efficient delivery of a pharmaceutically active amount of a taxane drug to a target site.

### Definitions

All technical and scientific terms used in this specification shall have the same meaning as commonly understood by persons of ordinary skill in the art to which the present invention pertains.

**Active ingredient** refers to an agent that is therapeutically effective.

**Amphiphile** or amphiphilic molecule is a molecule, which comprises a water-soluble (hydrophilic) and an lipid-soluble (lipophilic) part.

An **anionic amphiphile** refers to an amphiphile having a moiety that bears a negative net charge, preferably in a pH range of between 3 and 9, more preferably in a pH range of between 5 and 8.

Associating a negatively charged compound with a cationic amphiphile means binding of said compound to the cationic organic molecule by electrostatic and/or amphiphilic forces. The resulting composition is preferably characterized by a cationic/anionic charge ratio close to 1:1, but also charge ratios of from > 2:1 to 1:2, e.g. 1.5:1, may be used depending on the nature of the molecules.

**Cancer** refers to the more common forms of cancers such as bladder cancer, breast cancer, colorectal cancer, endometrial cancer, head and neck cancer, leukaemia, lung cancer, lymphoma, melanoma, non-small-cell lung cancer, ovarian cancer, prostate cancer and to childhood cancers such as brain stem glioma, cerebellar astrocytoma, cerebral astrocytoma, ependymoma, Ewing's sarcoma/family of tumors, germ cell tumor, extracranial, hodgkin's disease, acute lymphoblastic leukaemia, acute myeloid, liver cancer, medulloblastoma, neuroblastoma, non-hodgkin's lymphoma, osteosarcoma/malignant fibrous histiocytoma of bone, retinoblastoma, rhabdomyosarcoma, soft tissue sarcoma, supratentorial primitive neuroectodermal and pineal tumors, unusual childhood cancers, visual pathway and hypothalamic glioma, Wilms' Tumor and other childhood kidney tumors and to less common cancers including acute lymphocytic leukaemia, adult acute myeloid leukaemia, adult non-hodgkin's lymphoma, brain tumor, cervical cancer, childhood cancers, childhood sarcoma, chronic lymphocytic leukaemia, chronic myeloid leukaemia, esophageal cancer, hairy cell leukaemia, kidney cancer, liver cancer, multiple myeloma, neuroblastoma, oral cancer, pancreatic cancer, primary central nervous system lymphoma, skin cancer, small-cell lung cancer.

**Carrier** refers to a diluent, adjuvant, excipient, or vehicle with which a diagnostic or therapeutic agent is administered. The term also refers to one or several pharmaceutically acceptable component(s) that contains, complexes or is otherwise associated with an active ingredient to facilitate the transport of such an agent to its intended target site. Carriers include those known in the art, such as liposomes, polymers, lipid complexes, serum albumin, antibodies, cyclodextrins and dextrans, chelates, and other supra-molecular assemblies.

**Cationic** refers to a compound property that means positively charged, preferably in a pH range of between 3 and 9, more preferably in a pH range of between 5 and 8.

A **cationic amphiphile** refers to an amphiphile having a moiety that bears a positive net charge, preferably in a pH range of between 3 and 9, more preferably in a pH range of between 5 an 8. A cationic non-single chain amphiphile is a molecule bearing a positively charged moiety which comprises at least two hydrophobic hydrocarbon chains of at least 6, preferably of at least 8, more preferably of at least 10 and most preferably of at least 12 carbons and/or which comprises hydrophobic cyclic structures including the sterol structure such as cholesterol.

The term **"cationic lipid"** is used herein to encompass any lipid of the invention (as defined below) that is cationic, preferably in a pH range of between 3 and 9, more preferably in a pH range of between 5 and 8. The lipid will be determined as being cationic when the lipid has a positive net charge as measurable by instrumentation utilized at the time of the measurement. If the cationic lipid contains fatty acid moieties, they could be 12-24 carbons in length, each containing up to 6 unsaturated C-C bonds (double bonds), and linked to the backbone by ester and/or ether linkages; there could also only be one fatty acid chain linked to the backbone. When there is more than one fatty acid moiety linked to the backbone, the fatty acid moieties could be different (asymmetric).

**Cationic liposomes** are prepared from the cationic lipids themselves or in admixture with other lipids, particularly neutral lipids such as cholesterol; 1,2-diacyl-sn-glycero-3-phosphoethanolamines; 1,2-diacyl-sn-glycero-3-phosphocholines; natural lipids such as egg yolk phosphatidyl choline (PC), and the like; synthetic mono-and diacyl-phosphoethanolamines. The admixed phospholipids are not limited to dioleoyl fatty acid chains and the phospholipid may also have two different fatty acid chains. In addition, asymmetric fatty acids, both synthetic and natural, and mixed formulations, for the above fatty acid (diacyl) derivatives may also be included. A cationic liposome can comprise an excess of cationic lipids of at least 20 %, preferably at least 30 mol% and most preferably at least 40 mol% in the outer molecular layer. For further definition see also definition of "colloidal nanoaggregate having an overall cationic charge" and "liposome".

**Colloids** or colloidal particles are particles or molecular aggregates dispersed in a medium in which they are insoluble and have a size between about 5 nm and 5000 nm.

**Colloidal nanoaggregate having an overall positive charge** as used herein refers to a colloidal nanoaggregate comprising the composition of the invention and having an excess of positively charged molecules in the outer molecular layer. The nanoaggregate can comprise an excess of cationic lipids of at least 20 %, preferably at least 30 mol% and most preferably at least 40 mol%. As an example, if the nanoaggregate contains 10 mol% negatively charged lipids or negatively charged taxane derivatives the amount of positively charged lipid has to be at least 30 %, preferably at least 40 mol% and most preferably at least 50 mol% in order to fulfill the charge requirements.

**Cryoprotectant** is a substance that helps to protect a species from the effect of freezing, as for example during lyophilization.

**Derivative** is a compound derived from some other compound while maintaining its general structural features. For example, a derivative of a taxane drug with antimitotic activity that behaves as a prodrug, which itself may or may not have antimitotic activity. In general, derivatives may be obtained for example by chemical functionalization or derivatization.

**Disease characterized by enhanced angiogenic activity** refers to processes such as tissue inflammation, arthritis, asthma, macular degeneration, tumor growth, and diabetic retinopathy.

**Drug** as used herein may be a pharmaceutically acceptable compound which is pharmacologically active and/or physiologically active substances.

**Emulsion** or microemulsion refers to a system containing two immiscible liquids in which one is dispersed in the form of very small globules (internal phase) throughout the other (external phase). Emulsion or microemulsion can be a colloidal dispersion (emulsion particles or emulsion droplets) of two immiscible liquids (e.g., a liquid-liquid dispersion).

**Homogenization** refers to a physical process that achieves a uniform distribution between several components. One example is high-pressure homogenization.

The term **lipid** is used in its conventional sense as a generic term encompassing fats, lipids, alcohol-ether soluble constituents of protoplasm, which are insoluble in water. Lipids compose the fats, fatty oils, essential oils, waxes, steroid, sterols, phospholipids, glycolipids, sulpholipids, aminolipids, chromolipids, and fatty acids. The term encompasses both naturally occurring and synthetic lipids. Preferred lipids in connection with the present invention are: steroids and sterol, particularly cholesterol, phospholipids, including phosphatidyl and phosphatidylcholines and phosphatidylethanolamines, and sphingomyelins. If the lipid contains fatty acid moieties, they can be about 12-24 carbon chains in length, containing up to 6 double bonds, and linked to the backbone, the fatty acids can be different (asymmetric), or there may be only 1 fatty acid chain present, e.g., lysolecithins. Mixed formulations are also possible, particularly when the non-cationic lipids are derived from natural sources, such as lecithins (phosphatidylcholines) purified from egg yolk, bovine heart, brain, or liver, or soybean.

**Liposome** is a microscopic spherical membrane-enclosed vesicle (about 50-2000 nm diameter) made artificially in the laboratory. The term "liposome" encompasses an aqueous compartment enclosed by a lipid bilayer. Liposomes are also referred to as lipid vesicles. In order to form a liposome the lipid molecules comprise elongated non polar (hydrophobic) portions and polar (hydrophilic) portions. The hydrophobic and hydrophilic portions of the molecule are preferably positioned at two ends of an elongated molecular structure. When such lipids are dispersed in water they form spontaneously bilayer membranes referred to as lamellae. The lamellae are composed of two mono layer sheets of lipid molecules with their non-polar (hydrophobic) surfaces facing each other and their polar (hydrophilic) surfaces facing the aqueous medium. Liposomes are formed by bilayer forming lipids, i.e. lipids where the polar and non polar component claim similar volume in space (e.g. PC lipids). In addition, non-bilayer forming lipids (e.g. cholesterol or stearyl amine) can be added (50 mol%). Typically, a small quantity (( 10 mol%) of a charged amphiphile or lipid is added to ensure colloidal stability. The membranes formed by the lipids enclose a portion of the aqueous phase in a manner similar to that of a cell membrane enclosing the contents of a cell. Thus, the bilayer of a liposome has similarities to a cell membrane without the protein components present in a cell membrane. As used in connection with the present invention, the term liposome includes multilamellar liposomes, which generally have a diameter in the range of about 400 nm to about 5 æm and are comprised of anywhere from two to hundreds of concentric lipid bilayers alternating with layers of an aqueous phase, and also includes unilamellar vesicles which are comprised of a single lipid layer and generally have a diameter in the range of about 20 to about 500 nm, preferably about 50 to about 300 nm and most preferably about 100 to about 250 nm, which vesicles can be produced by subjecting multilamellar liposomes to ultrasound, by extrusion under pressure through membranes having pores of defined size, or by high pressure homogenization. Preferred liposomes are be unilamellar vesicles which have a single lipid bilayer, and a diameter in the range of about 25-400 nm.

**Lysolipid** is a lipid where one fatty acid ester has been cleaved resulting in a glycerol backbone bearing one free hydroxyl group.

**Lysophospholipid** is a phospholipid where one fatty acid ester has been cleaved resulting in a glycerol backbone bearing one free hydroxyl group.

**Micelle** is an association of amphiphiles in an aqueous solution characterized by an arrangement of the amphiphiles where the hydrophobic components are pointed outwards. A micelle does not have an aqueous interior compartment.

**Modifying** refers to making partial changes of the chemical structure of a compound.

**Nanoaggregate** is an assembly on a nanometer to micrometer scale assembled from smaller particles held together by attractive physical forces.

**Nanoparticle** is an entity in the dimensions of nanometers to micrometers.

**Negatively Charged Lipids** are lipids that have a negative net charge, preferably in a pH range of between 3 and 9, more preferably in a pH range of between 5 and 8, such as phosphatidic acid, phosphatidylserine, phosphatidylglycerol, phosphatidylinositol (not limited to a specific sugar), fatty acids, sterols containing a carboxylic acid group. The fatty acids linked to the glycerol backbone are not limited to a specific length or number of double bonds. Neutral phospholipids may also have two different fatty acids.

A **neutral amphiphile** refers to an amphiphile having a polar non-charged moiety or a zwitterionic (neutral net charge) moiety, preferably in a pH range of between 3 an 9, more preferably in a pH range of between 5 an 8.

**Neutral Lipids** are lipids that have a neutral net charge, preferably in a pH range of between 3 and 9, more preferably in a pH range of between 5 and 8, such as cholesterol, 1,2-diacyl-sn-glycero-3-phosphoethanolamine, including but not limited to dioleoyl (DOPE, a large family of derivatives is available form Avanti Polar Lipids), 1,2-diacyl-glycero-3-phosphocholines (a large family of derivatives is available form Avanti Polar Lipids), Sphingomyelin.

A **non-single chain amphiphile** is a molecule which comprises at least two hydrophobic hydrocarbon chains of at least 6, preferably of at least 8, more preferably of at least 10 and most preferably of at least 12 carbons and/or which comprises hydrophobic cyclic structures including the sterol structure such as cholesterol.

**Particle diameter** refers to the size of a particle. In the present patent, in order to determine experimentally particle diameters of nanoparticles and liposomes, dynamic light scattering (DLS) using a Malvern Zetasizer 1000 or 3000 (Malvern, Herrenberg, Germany) was performed. For quantitative data analysis, in all cases parameters (refractive index, density, viscosity) of pure water were taken, even if the aqueous phase contained cryoprotectants to a certain extend. Therefore, for the absolute numbers, a certain systematic deviation with respect to literature data may have to be taken into account. Particle diameter is expressed as Z_{average}. The Z_{average} value is obtained by fitting all measured particle sizes to a Gaussian distribution and taking the mean of that distribution (ISO 13321).

**Pegylated** lipid is a lipid bearing one ore more polyethylene glycol (PEG) residues such as DOPE-PEG where the PEG chain has a molecular weight between 350 and 5000 Da (a large family of PEG derivatives is available form Avanti Polar Lipids).

**Phospholipid** is a lipid comprising a glycerol backbone, a phosphate group and one or more fatty acids which are bound to the glycerol backbone by ester bonds.

**Pl or polydispersity index** is a dimensionless measure of the broadness of the size distribution (ISO 13321).

**Positively Charged Lipids** is a synonym for cationic lipids (for definition see definition of "cationic lipids").

**Reversible** refers to a chemical process that can be changed in such a way that the process is reversed.

**Sterol** is a steroid alcohol. Steroids are derived from the compound called cyclopentanoperhydrophenanthrene. Well-known examples of sterols include cholesterol, lanosterol, and phytosterol.

**Taxane drug** with antimitotic activity as used herein refers to a class of agents having a mechanism of microtubule action and having a structure that includes the taxane ring structure. One of the most prominent representatives of this group is paclitaxel. But also other analogues or derivatives belonging to this of this drug family are known such as for example docetaxel, 10-desacetyl analogues of paclitaxel, 7-epi-taxol and 3'-N-desbenzoyl-3'-N-t-butoxycarbonyl analogues of paclitaxel. (see also W094/07882,W094/07881,W094/07880,W094/07876,W093/23555, W093/10076; US5,294,637; US5,283,253; US5,279,949; US5,274,137; US5,202,448; US5,200,534; US5,229,529; and EP590,267) A taxane drug should be understood to refer to not only the common chemically available paclitaxel, but analogues (e. g., docetaxel or other taxane derivatives, as noted above) and paclitaxel conjugates (e. g., paclitaxel-PEG, paclitaxel-dextran or paclitaxel-xylose or other derivatives that were synthesized starting from paclitaxel itself).

**Therapeutic agent** refers to a species that reduces the extent of the pathology of a disease such as cancer. Such a compound may, for example, reduce primary tumor growth and, preferably, the metastatic potential of a cancer. Alternatively, such a compound may reduce tumor vascularity, for example either by decreasing microvessel size or number or by decreasing the blood vessel density ratio.

**Virtually free** of a species is defined as not detectable by HPLC.

**Zeta potential** refers to a surface potential of a particle such as a colloidal particle measured with an instrument such as a Zetasizer 3000 using Laser Doppler micro-electrophoresis under the conditions specified. The zeta potential describes the potential at the boundary between bulk solution and the region of hydrodynamic shear or diffuse layer. For the present invention, zeta potential measurements were performed with a Malvern Zetasizer 3000 (Malvern, Herrenberg, Germany).

### Description of Prior Art

Taxane drugs are a class of antimitotic agents having a characteristic ring structure. One of their most prominent representatives is Paclitaxel. Paclitaxel is a natural diterpenoid isolated from the bark of the Pacific yew tree (Taxus brevifolia). Paclitaxel has been approved for treatment of patients with advanced ovarian cancer, breast cancer, Kaposi's Sarcoma and non-small cell lung cancers.

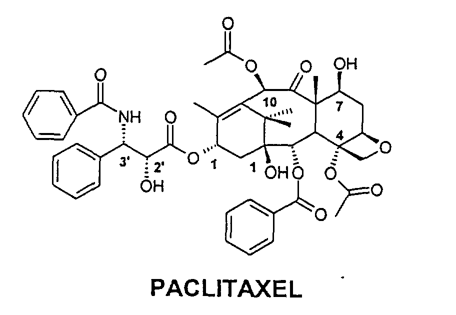

Paclitaxel binds to microtubule and promotes tubulin polymerization and stabilizes the assembled microtubules. Paclitaxel blocks the cell cycle at prophase resulting in an accumulation of cells in the G₂/M phase [Horwitz S.B. Ann. Oncol. 1994, 5, 3-6].

Unfortunately, taxane drugs exhibit a very low water solubility that limits its administration to patients. Various reports have been published on water solubility of paclitaxel revealing values between 10 µg/ml and 0.05 µg/ml [Damen et al. Bioorg. Med. Chem. 2000, 8, 427-432; Wenk et al. J. Pharm. Sci. 1996, 85, 228-231]. Due to this unfavorable intrinsic property paclitaxel is formulated using a 1:1 mixture of Cremophor® EL (a polyethoxylated castor oil) and ethanol/water. However, various severe side reactions, such as hypersensitivity and hypertensive reactions, nephrotoxicity and neurotoxicity, have been reported in patients due to Cremophor® EL formulation [Dorr R.T. Ann. Pharmacother. 1994, 28, 511-514].

The formulation problem of paclitaxel has been addressed by the development of more water soluble derivatives that act as prodrugs of paclitaxel. The prodrug strategy is based on a reversible chemical modification and thereby changed physicochemical properties leading to improved aqueous solubility. However, for each modification pharmacological parameters need to be adjusted. The necessary release of the active paclitaxel drug is usually observed by enzymatic mechanisms or hydrolysis under physiological conditions.

Recently, syntheses and biological data of new paclitaxel derivatives with improved water solubility due to the linkage of a polar substituent either to the C2'- or the C7-hydroxyl group were published. Most commonly, the polar substituent has been attached via an ester, carbonate or carbamate functionality.

Negatively charged derivatives have been synthesized such as C2'- or C7-succinyl paclitaxel and C2'-glutaryl paclitaxel [US4942184, Deutsch et al. J. Med. Chem. 1989, 32, 788-792]. Compared to the mono and bis-succinate which are not water soluble (similar to paclitaxel) the sodium salts of the acids show improved aqueous solubility. Damen et al. [Damen et al. Bioorg. Med. Chem. 2000, 8, 427-432] reported negatively charged C2'- and C7-malyl paclitaxel derivatives with aqueous solubility of 0.2 mg/ml (C2' derivative) and 0.3 mg/ml (C7 derivative). In case of the C2'-malyl derivative, paclitaxel release properties were found dependent on whether the compound was synthesized as sodium salt (no paclitaxel after 48 h) or as free acid (50% after 24 h). Moreover, the C7-malyl analogue showed an lC₅₀ value higher than that of the unmodified drug by a factor of at least 100 (MCF-7 cells) indicating that this modification increased water solubility but at the expense of biological activity. The C2'-malyl analogue showed comparable lC₅₀ values to paclitaxel but the determined aqueous solubility is still too low in order to reach an administrable injection volume to realize a suitable dosage treatment.

Neutral derivatives in which paclitaxel is linked to a polyethylene glycol moiety were synthesized by Greenwald et al. [Greenwald et al. J. Med. Chem. 1993, 3, 1761-1766]. The C2'- and C7-PEG derivatives were highly water soluble. The C2'-analogue had in vitro and in vivo activities to the same extent as paclitaxel (P388 model).

However, in these new compounds paclitaxel is rapidly released in aqueous solutions and precipitates. This limits formulation and storage of these paclitaxel analogues.

In order to enhance aqueous solubility liposomes have been used to formulate taxane drugs. However, attempts to formulate with liposomes consisting of neutral or anionic lipids have been of little success due to a low drug to lipid ratio [Sharma et al. Cancer Lett. 1996, 107, 265-272]. The low drug to lipid ratio is due to the limited solubility of the taxane drugs in the lipid membrane. At lipid to drug ratios higher than about 3 mol% free paclitaxel precipitates out of the liposomal formulation [Sharma et al. Pharm. Res. 1994, 11, 889-895].

Recently, it has been shown that positively charged liposomes based on cationic lipids which form lipid bilayers represent not only another variety of liposomal carrier system but also target angiogenic endothelium at a tumor site when injected into the blood circulation [WOO1/17508, US5837283, US6120799]. However, cationic liposomes can be loaded only up to 3 mol% paclitaxel as shown by Campbell et al. [Campbell et al. J. Pharm. Sci. 2001, 1091-1105]. Liposomes with a content higher than 3 mol% paclitaxel were thermodynamically instable resulting in extensive drug precipitation after 3 hours storage.

In another approach, Ceruti et al. [Ceruti et al. J. Control. Release 2000, 63, 141-153] reported data on encapsulating different water-soluble paclitaxel derivatives into the aqueous compartment of PC-containing liposomes. For example, using C2'-succinyl paclitaxel they found a maximum final drug to lipid ratio of only 3-5 mol% when PC-containing liposomes were used stabilized with 10 mol% charged lipid (usually PG or stearyl amine). However, the C2'-succinyl paclitaxel containing liposomes showed a reduced encapsulation stability (85 % of liposomal paclitaxel derivative after 1 week).

Thus, the problem underlying the present invention was to provide a composition of a taxane drug and a formulation thereof with a high drug to lipid ratio and high physicochemical stability for most efficient delivery of a pharmaceutically active amount of a taxane drug with antimitotic activity to a target site.

### Detailed Description

The solution to the problem above is achieved according to the invention by providing the embodiments characterized in the claims.

Accordingly, the present invention relates to a composition comprising a derivative of a taxane drug with antimitotic activity which has a negative net charge (anionic derivative) and an amphiphile which has a positive net charge (cationic amphiphile), preferably in a pH range of between 3 an 9, more preferably in a pH range of between 5 an 8. The novel composition of an anionic taxane drug derivative and a cationic amphiphile overcomes problems of formulating a lipophilic taxane drug by its new physicochemical properties. The inventive composition is stabilized by electrostatic forces and/or amphiphilic interactions between drug and amphiphile.

### The advantages of the present invention can be described as follows:

The taxane drug in the novel composition is present in a stabilized form. Stabilization is effected by anionic derivatization of the taxane drug and additionally by formation of a composition with the positively charged amphiphile. By means of this stabilization formulations are obtainable which are virtually free from degradation products and from precipitates comprising the anionic taxane drug derivative component. In contrast thereto, these undesired products are formed in known paclitaxel formulations [Campbell et al. J. Pharm. Sci. 2001, 1091-1105].

The amphiphilic characteristics of the novel composition allow the manufacture of stable formulations comprising high drug amounts for most efficient drug delivery. This stabilization may be improved by additional interactions (amphiphilic interactions) between the disclosed composition and other amphiphiles. It was found, however, that single chain amphiphiles, such as stearyl amine, at higher concentrations than 10-15 mol % significantly lower the physical stability of e.g. liposomal formulations. Thus, in a preferred embodiment cationic non-single chain amphiphiles are used for the manufacture of the composition.

The optimized physicochemical properties of the composition are responsible for more efficient delivery of the taxane drug to the target site in vivo. The advantages of the stabilized formulations of the composition result in improved treatment efficacy, such as minimized side effects after systemic administration.

The present invention is not limited to paclitaxel or docetaxel but can also be applied to other taxane drugs with antimitotic activity or even to compounds having the common pharmacophore, as e.g. for recently discovered epothilones, which share a common structure-activity relationship profile [Giannakakou et al. PNAS 2000, 2904-2909]. The common pharmacophore includes taxane, epothilone and sarcodictyin drugs which share a common antimitotic activity on the same molecular basis and structure by identifying an overlap of the binding motive at the target sites of tubulin polymers. Drugs with the same mode of action particularly include taxane drugs, epothilones A, B, C and D the marine sponge-derived discodermolide and the coral- derived eleutherolbins/sarcodictyins.

Preferably, the taxane drug is chemically modified with at least one moiety that bears a negative net charge (negatively charged moiety). The negatively charged moiety may be covalently linked to the taxane drug via reacting the C7- and/or the C2'-hydroxyl group and/or another reactive functional group of the taxane molecule. The C2'-hydroxyl group is preferred. In a preferred embodiment the negatively charged moiety is linked to the taxane via an ester or an ether bond and in an even more preferred embodiment via an ester bond.

The negatively charged moiety can be selected from molecules having at least one acidic group such as carboxylic acids, e.g. selected from lactic acid, citric acid, succinic acid, glutaric acid, maleic acid, fumaric acid, malonic acid or adipic acid. The invention, however, contemplates the use of other acidic groups, e.g. acids containing a heteroatom such as sulfur or phosphorus or others. The acids are selected such that after coupling with the taxane drug at least one free unreacted acid group remains. The bond between taxane and negatively charged moiety should be chemically stable during drug formulation, application and delivery to the biological target site. On the other hand, the bond should be also cleavable under predetermined conditions in order to enable a release of the taxane drug with antimitotic activity which should occur at the target site in a therapeutically efficient degree. Preferably, the release should occur substantially quantitatively. Thus the chemical modification of the taxane drug is not carried out to increase the water solubility but to provide a drug exhibiting increased amphiphilic interactions with the cationic amphiphile. As disclosed above, further stabilization of the novel composition is gained from electrostatic forces between the anionic taxane derivative and the cationic amphiphile.

In a preferred embodiment the inventive composition is virtually free of the unmodified taxane drug.

The cationic amphiphile of the present invention may be selected from lipids, lysolipids or pegylated lipids having a positive net charge. Useful cationic lipids thereby include:

DDAB, dimethyldioctadecyl ammonium bromide; N-[1-(2,3-dioleoyloxy)propyl]-N,N,N-trimethyl ammonium methylsulfate (DOTAP); 1,2-diacyloxy-3-trimethylammonium propanes, (including but not limited to: dioleoyl, dimyristoyl, dilauroyl, dipalmitoyl and distearoyl; also two different acyl chain can be linked to the glycerol backbone); N-[1-(2,3-dioloyloxy)propyl]-N,N-dimethyl amine (DODAP); 1,2-diacyloxy-3-dimethylammonium propanes, (including but not limited to: dioleoyl, dimyristoyl, dilauroyl, dipalmitoyl and distearoyl; also two different acyl chain can be linked to the glycerol backbone); N-[1-(2,3-dioleyloxy)propyl]-N,N,N-trimethylammonium chloride (DOTMA); 1,2-dialkyloxy-3-dimethylammonium propanes, (including but not limited to: dioleyl, dimyristyl, dilauryl, dipalmityl and distearyl; also two different alkyl chain can be linked to the glycerol backbone); dioctadecylamidoglycylspermine (DOGS), 3β-[N-(N',N'-dimethylaminoethane)carbamoyl]cholesterol (DC-Chol); 2, 3-dioleoyloxy-N-(2-(sperminecarboxamido)-ethyl)-N,N-dimethyl-1-propa naminium trifluoro-acetate (DOSPA); β-alanyl cholesterol; cetyl trimethyl ammonium bromide (CTAB); diC14-amidine; N-tert-butyl-N'-tetradecyl-3-tetradecylaminopropionamidine; 14Dea2; N-(alpha-trimethylammonioacetyl)didodecyl-D-glutamate chloride (TMAG); O,O'-ditetradecanoyl-N-(trimethylammonioacetyl)diethanolamine chloride; 1,3-dioleoyloxy-2-(6-carboxy-spermyl)-propylamide (DOSPER); N,N,N',N'-tetramethyl-N,N'-bis(2-hydroxylethyl)-2,3-dioleoyloxy-1,4-buta nediammonium iodide; 1-[2-(acyloxy)ethyl]2-alkyl(alkenyl)-3-(2-hydroxyethyl)-imidazolinium chloride derivatives as described by Solodin et al. (1995) Biochem. 43:13537-13544, such as 1-[2-(9(Z)-octadecenoyloxy)ethyl]-2-(8(Z)-heptadecenyl-3-(2-hydroxyethyl )imidazolinium chloride (DOTIM), 1-[2-(hexadecanoyloxy)ethyl]-2-pentadecyl-3-(2-hydroxyethyl)imidazolinium chloride (DPTIM), 2,3-dialkyloxypropyl quaternary ammonium compound derivatives, contain a hydroxyalkyl moiety on the quaternary amine, as described e.g. by Felgner et al. [Felgner et al. J. Biol. Chem. 1994, 269, 2550-2561] such as: 1,2-dioleoyl-3-dimethyl-hydroxyethyl ammonium bromide (DORI), 1,2-dioleyloxypropyl-3-dimethyl-hydroxyethyl ammonium bromide(DORIE),1,2-dioleyloxypropyl-3-dimetyl-hydroxypropyl ammonium bromide (DORIE-HP), 1,2-dioleyloxypropyl-3-dimethyl-hydroxybutyl ammonium bromide (DORIE-HB), 1,2-dioleyloxypropyl-3-dimethyl-hydroxypentyl ammonium bromide (DORIE-Hpe), 1,2-dimyristyloxypropyl-3-dimethyl-hydroxylethylammonium bromide (DMRIE), 1,2-dipalmityloxypropyl-3-dimethyl-hydroxyethyl ammoniumbromide(DPRIE),1,2-disteryloxypropyl-3-dimethyl-hydroxyethyl ammonium bromide (DSRIE); cationic esters of acyl carnitines as reported by Santaniello et al. [US5498633].

In a preferred embodiment the cationic amphiphile is selected from a quaternary ammonium salt such as a N-[1-(2,3-diacyloxy)propyl]-N,N,N-trimethyl ammonium salt, wherein each acyl group preferably contains at least 6 carbon atoms, and a pharmaceutically acceptable counter anion of the quaternary amino compound is selected, e.g. from the group consisting of chloride, bromide, fluoride, iodide, nitrate, sulfate, methyl sulfate, phosphate, acetate, benzoate, citrate, glutamate or lactate.

Further, amphiphiles having negative and/or neutral net charges (anionic and/or neutral amphiphiles) can be incorporated into the composition. These can be selected from sterols or lipids such as cholesterol, phospholipids, lysolipids, lysophospholipids, sphingolipids or pegylated lipids with a negative or neutral net change. Useful anionic and neutral lipids thereby include: Phosphatidic acid, phosphatidylserine, phosphatidylglycerol, phosphatidylinositol (not limited to a specific sugar), fatty acids, sterols containing a carboxylic acid group, cholesterol, 1,2-diacyl-sn-glycero-3-phosphoethanolamine, including but not limited to dioleoyl (DOPE, a large family of derivatives is available form Avanti Polar Lipids), 1,2-diacyl-glycero-3-phosphocholines (a large family of derivatives is available form Avanti Polar Lipids), sphingomyelin. The fatty acids linked to the glycerol backbone are not limited to a specific length or number of double bonds. Phospholipids may also have two different fatty acids. In a preferred embodiment the neutral amphiphile is diacylphosphatidylcholine.

The resulting composition is highly soluble in any kind of colloidal nanoaggregate such as emulsion droplets, liposomes, micelles or nanoparticles due to the improved stabilizing interactions between the described composition and other cationic, anionic and/or neutral amphiphiles.

Thus, a further aspect of the present invention relates to a colloidal nanoaggregate comprising the inventive composition and having an overall positive charge. In this context overall positive charge means an excess of positively charged molecules in the molecular surface layer of the colloidal nanoaggregate. In a preferred embodiment, the inventive nanoaggregate comprises an excess of cationic lipids of at least 20 mol%, preferably at least 30 mol% and most preferably at least 40 mol%. As an example, if the nanoaggregate contains 10 mol% negatively charged lipids or negatively charged taxane derivatives the amount of positively charged lipid has to be at least 30 %, preferably at least 40 mol% and most preferably at least 50 mol% in order to fulfil these charge requirements. Measurements of the zeta potential can be used to determine the net charge of the colloidal nanoaggregates. Usually, zeta potentials in the range between 25 mV and 100 mV, preferably between 35 mV and 70 mV, in about 0.05 mM KCI solution at about pH 7.5 at room temperature are preferred. However, zeta potential values lower than 25 mV are suitable, when colloidal nanoaggregates are formulated with pegylated lipids.

The amount of a taxane derivative in the cationic colloidal nanoaggregates of the present invention is equal or less than about 50 mol%, e.g. from about 1 to 50 mol%, particularly from about 5 to 30 mol%, and more particularly from about 8 to 20 mol%. The amount of the taxane derivative may be determined by HPLC.

Thus the invention allows a significant increase in the amount of taxane drug compared to prior art formulations of e.g. paclitaxel where a maximum amount of about 3 mol% has been reached. Besides the increased loading of lipid membranes also an increased stability of the taxane drug is observed. In contrast to formulations of the unmodified taxane drug, formulations containing the inventive composition protect the taxane drug against any kind of chemical degradation leading to impurities such as 7-epitaxol, 10-desacetyltaxol, baccatin-lll or other degradation products that have been found in known liposomal formulations containing paclitaxel or other taxane drugs.

The nanoaggregate of the invention can be present in different supramolecular assemblies with different size ranges. Thus, in further preferred embodiments, the nanoaggregate is present as an emulsion droplet, a liposome, a micelle or a nanoparticle. The particle diameter of the nanoaggregate can range from about 5 nm to about 1000 nm, preferably between 25 and 500 nm and more preferably between 100 to 300 nm.

The taxane drug derivative does not partition substantially from the colloidal nanoaggregate within a time period of at least 0.5 h, generally at least about 1 h, generally at least about 2 h, usually at least about 24 h, usually at least about 48 hours, at a temperature between about 4 °C and 25 °C. In addition, the taxane drug or the respective taxane drug derivative does not form crystals that would be detected under a microscope (100-fold magnification). Higher proportions of the taxane drug derivatives as have been disclosed above can be incorporated as long as the taxane drug derivative does not substantially partition from the colloidal nanoaggregate and/or contains substantially no taxane crystals from the taxane drug or from the taxane drug derivative. "Substantially no taxane crystals" as used herein generally relates to less than about 10 %, usually less than about 5 %, usually less than about 2 %, typically less than about 1 %, and preferably less than about 0.5 % of the taxane drug or of the taxane drug derivative in the inventive nanoaggregate in the form of crystals. A colloidal nanoaggregate in which the taxane drug derivative does not substantially partition is one in which generally less than about 20 %, usually less than 10 %, usually less than about 5 %, typically less than about 1 %, and preferably less than about 0.5 % of the taxane drug derivative has partitioned from the inventive nanoaggregate.

Preferred nanoaggregates of the present invention are cationic liposomes comprising DOTAP, DODAP, analogues of DOTAP or DODAP or any other cationic lipid in an amount of at least 20+x mol%, preferably of at least 30+x mol%, more preferably of at least 40+x mol% and further DOPC, pegylated lipids or any other lipid that has a neutral net charge in an amount of up to 80-2x mol%, preferably up to 70-2x mol% and most preferably up to 60-2x mol% where x mol% is the amount of the taxane drug derivative and an optionally present negatively charged amphiphile.

It is a further objective of the present invention to provide a cationic colloidal nanoaggregate such as a liposome which can be freeze-dried (lyophilized), stored for an extended time period and then reconstituted by adding liquid, e.g. water to the dry lyophilisates. Reconstitution of the lyophilisates is performed when and where they are to be used without losing a substantial portion of their contents during the freeze-drying process (lyophilization), storage and reconstitution step. To achieve the latter one or more protective agents such as cryoprotectants can be present. Thus, preferably the nanoaggregate further comprises a cryoprotectant. The cryoprotectant may be selected from sugars or alcohols or particularly sugar alcohols or combinations thereof. Preferably, the cryoprotectant is selected from trehalose, maltose, sucrose, glucose, lactose, dextran, mannitol, sorbitol and combinations thereof.

In accordance with other aspects of the invention, a pharmaceutical preparation comprising a pharmaceutically effective amount of the inventive composition or an inventive colloidal nanoaggregate together with a pharmaceutically acceptable carrier, diluent and/or adjuvant is provided.

The nanoaggregates of the present invention are produced by a method comprising the following 3 steps:
a) providing a derivative of a taxane drug with antimitotic activity which has a negative net charge (anionic derivative) and
b) associating said derivative with a cationic amphiphile which has a positive net charge (cationic amphiphile) and
c) optionally admixing at least one further amphiphile in step b) which has a positive, negative and/or neutral net charge (cationic, anionic and/or neutral amphiphile) forming a colloidal nanoaggregate with an overall cationic charge, as described above.

In a preferred embodiment steps b) and c) can be performed by various methodologies, well known to ordinarily skilled artisans. Sonication, vortexing, mechanical stirring, static mixing, homogenization, solvent injection, lipid film method, microfluidization, colloid mills and/or pressure emulsifiers can be used to prepare nanoaggregates.

The preferred inventive SuccTXL-DOTAP composition may be formed in-situ during the nanoaggregate preparation. Alternatively, the nanoaggregate may be preformed and subsequently incorporated into the composition.

The lipid film procedure comprises producing a lipid film of a cationic amphiphile, said derivative of a taxane drug and optionally at least one cationic, anionic and/or neutral amphiphile, drying and subsequently rehydrating.

In the solvent injection method, the lipid is dissolved in a solvent and the lipid-solvent solution is injected into an aqueous solution. The solvent may remain in the resulting aqueous solution or may be removed, e.g. by crossflow diafiltration or during lyophilization [See e.g., Deamer et al. Annals New York Academy of Sciences 1978, 308, 250-258]. In a preferred embodiment, the solvent injection procedure comprises adding an organic solution comprising a cationic amphiphile and optionally at least one anionic and/or neutral amphiphile and said derivative to an aqueous solution, wherein said organic solution comprises an organic solvent selected from methanol, ethanol, propanol, isopropanol, ethylene glycol, tetrahydrofuran, chloroform or diethylether or a mixture of these solvents. The aqueous solution may further comprise a cryoprotectant as described above.

In another preferred embodiment, the inventive method can further comprise at least one dialysis and/or at least one homogenization and/or optionally at least one sterile filtration and/or optionally a freeze drying and/or optionally a reconstitution step. Dialysis might be necessary to remove the organic solvent or another component from the mixture if the concentration of the solvent is too high and/or the solvent is detrimental to the stability/overall quality of the nanoaggregate. If the nanoaggregates do not have the desired size distribution, an additional homogenization step can be performed subsequently. This can be performed by extrusion through a membrane with defined pore size, high pressure homogenization, ultrasound treatment, high speed homogenization or any other homogenization technique well known in the art.

Angiogenesis associated diseases are dependent on blood supply. The local interruption of the vasculature will produce an avalanche of cell death. The vascular endothelium is in direct contact with the blood. It is contemplated that a variety of diseases including human diseases can be prevented and/or treated with the foregoing methods and compositions. In a preferred embodiment, a composition, a colloidal nanoaggregate or a pharmaceutical formulation as provided by the present invention can be used for preventing and/or treating a disease such as cancer, a variety of inflammatory diseases, diabetic retinopathy, rheumatoid arthritis, dermatitis, psoriasis, stomach ulcers, macular degeneration, hematogenous and solid tumors. In a further preferred embodiment, the invention can be applied for producing a medicament for preventing and/or treating solid tumors and their metastases such as bladder, brain, breast, cervical, colorectal, endometrial, head and neck or kidney cancer, leukemia, liver or lung cancer, lymphoma, melanoma, non-small-cell lung, ovarian, pancreatic or prostate cancer. Particularly, the targeted cells are endothelial cells.

A further objective of the present invention is producing a medicament for the selective delivery of a taxane drug with antimitotic activity to an angiogenic vascular target site, wherein delivery comprises the steps of
a) providing a composition and/or a colloidal nanoaggregate and/or a pharmaceutical composition of the invention,
b) administering said composition and/or colloidal nanoaggregate and/or pharmaceutical composition to a subject in need thereof,
c) allowing the composition and/or colloidal nanoaggregate and or pharmaceutical composition to associate with the target site and
d) releasing said taxane drug or said derivative at the target site.

Preferably, step d) comprises not only liberating said taxane derivative from the associated cationic amphiphile but also cleaving the said negatively charged moiety from the taxane drug, e.g. by hydrolysis of a covalent bond, to allow the taxane drug to effect its anti tumor activity.

In a preferred embodiment, the method comprises cellular uptake of the taxane drug or the taxane drug derivative. The uptake mechanism may comprise uptake of the colloidal nanoaggregate at the target site via endocytosis and/or fusion with the cell membrane followed by the release of the taxane drug or taxane drug derivative as described above (step d). An alternative mechanism is that the taxane drug or taxane drug derivative is released on the extracellular site of the cell membrane followed by permeation across the membrane to enter the cell.

The following examples should be illustrative only but are not meant to be limiting to the scope of the invention. Other generic and specific configurations will be apparent to those skilled in the art.

### Examples

### 1. Synthesis of Compositions of Derivatized Anionic Taxanes and Cationic Amphiphiles

### 1.1 Synthesis of Succinyl Paclitaxel

Succinyl paclitaxel (SuccTXL) was synthesized as shown in Figure 1 based on the procedure by Horowitz [US4942184] or Dosio [Dosio et al. J. Control. Release 1997, 47, 293-304].
Paclitaxel (33 mg, 0.0386 mmol) was dissolved in 0.5 ml of dry pyridine to which 7.7 mg of succinic anhydride (0.0772 mmol) and 0.5 mg (0.00386 mmol) of 4-dimethyl aminopyridine were added. The resulting solution was stirred for 3 h at room temperature. The product was purified by chromatography on a silica-gel 60 column to give 34.6 mg of succinyl paclitaxel. Rf (silica gel, TLC plates) was 0.42 in chloroform-methanol (90:10). Yield: 84 %.

### Structural Characterization:

The chemical structure of succinyl paclitaxel is confirmed by NMR, TLC and MS analysis. Analytical date are consistent with results reported in literature and purity is higher than 98% according to HPLC analysis. Some parameters of the applied HPLC method are outlined in Table 1:

**Table 1:**

| HPLC method for purifying succinyl paclitaxel | |
|---|---|
| Flow rate | 1 ml/min |
| Wavelength | 229 nm |
| Injected volume | 20 µl |
| Mobile phase | Acetonitril/THF/2 mM ammonium acetate buffer (pH 5), 32/12/56 (v/v/v), pH 4.7 (adjusted with acetic acid) |
| Column | LiChroCart 250-4, LiChrospher 60, RP-select B (Precolumn: 8/4 Lichrospher 100-5 C18) |
| Retention Time | Paclitaxel: 17-20 min succinyl paclitaxel: 7-10 min |

### 1.2. Synthesis of Other Anionic Derivatives of Paclitaxel and Succinyl-Docetaxel

Following the procedure published by Damen [Damen et.al. Bioorg. & Med. Chem. 2000, 8, 427-432] the 2'malyl paclitaxel is prepared in a 3-step reaction sequence starting from paclitaxel.

The procedure applied for the synthesis of succinyl paclitaxel is also used to synthesis 2'-adipyl paclitaxel. Instead of the succinyl anhydride adipyl chloride or any other activated form of adipic acid is reacted with paclitaxel.

In all cases analytical data are consistent with the chemical structures of the anionic derivatives of paclitaxel.

Docetaxel has been also derivatized yielding the 2'-succinyl docetaxel applying the same reaction conditions as described above. The reaction is performed with 80 % yield and product is isolated as a white powder. Analytic data (NMR, TLC and MS analysis) were consistent with the theoretical data and purity is found to be higher than 98 % (HPLC).

### 1.3 Synthesis of Succinyl Paclitaxel-DOTAP (SuccTXL-DOTAP)

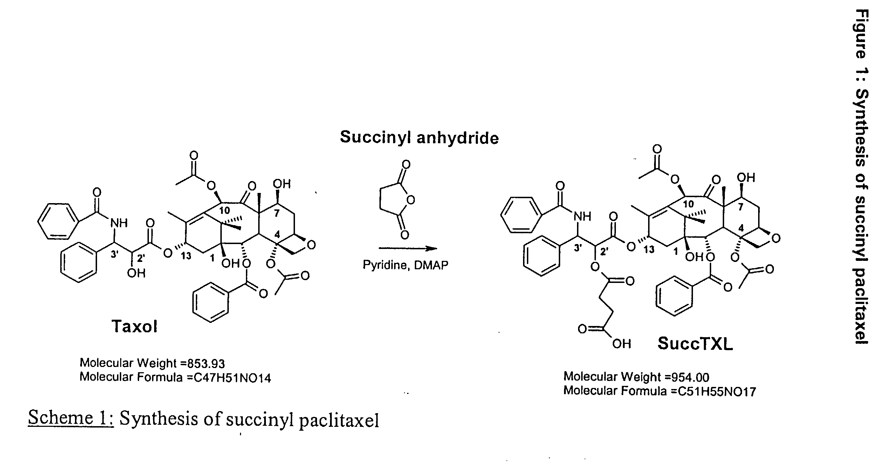

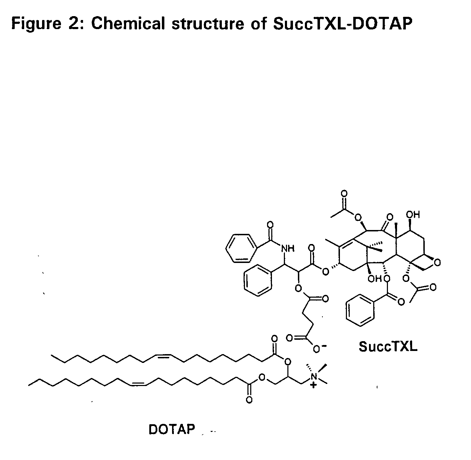

The SuccTXL-DOTAP as shown in Figure 2 may be formed by precipitation or crystallization from an organic solution of succinyl paclitaxel and DOTAP as follows:
A methanolic solution of succinyl paclitaxel and DOTAP (N-[1-(2,3-dioleoyloxy)propane]-N,N,N-trimethyl ammonium chloride) at equimolar concentration ratios is added to water resulting in a fine precipitation of SuccTXL-DOTAP. After evaporation of the solvent the remaining solid is dissolved in water and freeze-dried to give a white fine power of the SuccTXL-DOTAP.

Chemical analysis of the inventive SuccTXL-DOTAP composition:
¹NMR-spectroscopy (Bruker 400 MHz, CDCl₃:CD₃OD 3:1:TMS); DOTAP related signals: (ppm) (0.89 (t, J=7.0 Hz, 6H, CH₃), 1.23 (m, 40H; 4-7-und 12-17-CH₂), 1.65 (m, 4H; 3-CH₂), 2.02 (m, 8H; 8- und 11 -CH₂), 2.36 (m, 4H; 2-CH₂), 3.20 (s, 9H; N+(CH₃)₃), 3.62-3.80 (m, 2H; N-CHH, N-CHH), 4.05 (m, 1H; O-CHH), 4.47 (m, 1H; O-CHH), 5.34 (m, 4H, 9 and 10-CH), 5.60 (m, 1H; CH-O); succinyl paclitaxel related signals: (ppm) 1.11 (S, 3H, C17-H), 1.19 (s, 3H, C16-H), 1.62 (s, 3H, C19-H), 1.76 (s, 3H, C18-H), 2.2 (m, 2H, C14-H), 2.22 (s, 3H, C10-OAc), 2.43 (s, 3H, C4-OAc), 2.6 (m, 14H, 16H), 2.22 (s, 3H, OAc), 2.43 (s, 3H, OAc), 2.6 (m, 4H, CH CH ), 3.34 (d, 1H, C3-H), 4.17 (d, 1H, C20-H), 4.48 (d, 1H, C7-H), 4.96 (dd, 1H, C5-H), 5.51 (d, 1H, C29-H), 5.67 (d, 1H, C2-H), 6.21 (t, 1H, C13-H), 6.27 (s, 1H, C10-H), 7.07 (d, 1H, NH), 7.3-8.1 (m, 15H, arom.).
Elemental analysis data confirm the structure.

### 1.4. Synthesis of Further Succinyl Paclitaxel-Cationic Amphiphiles (SuccTXL-CA)

Compositions containing succinyl paclitaxel and other cationic amphiphiles are prepared based on the procedure as applied for SuccTXL-DOTAP. SuccTXL-cationic amphiphile compositions are prepared with an cationic amphiphile selected from the following list:

| | |
|---|---|
| DSTAP | N-[1-(2,3-distearyloxy)propane]-N,N,N-trimethyl ammonium chloride |
| DMTAP | N-[1-(2,3-dimyristoyloxy)propane]-N,N,N-trimethyl ammonium chloride |
| DODAP | N-[1-(2,3-dioleoyloxy)propane]-N,N-dimethyl amine |
| DSDAP | N-[1-(2,3-distearoyloxy)propane]-N,N-dimethyl amine |
| DMDAP | N-[1-(2,3-dimyristoyloxy)propane]-N,N-dimethyl amine |

In all cases ¹NMR-spectroscopic and elemental analysis data confirm the chemical structure of the respective product.

### 2. Liposomal Formulations of SuccTXL-DOTAP

Liposomes are formed by lipid film method or ethanol injection but also other methods can be applied. Liposome preparation employing the lipid film method and the ethanol injection are described in the following. The inventive SuccTXL-DOTAP composition is formed in-situ during the preparation. An alternative synthesis of the composition in a first step and subsequently formulating has been compared with the in-situ preparation without showing a difference in the result.

### 2.1. Liposome Preparation via Lipid Film Method

Liposomal formulations comprising the SuccTXL-DOTAP are prepared using the lipid film method as follows: The lipids and succinyl paclitaxel are dissolved in chloroform in a round bottom flask. The flask is then rotated under vacuum (100 to 200 mbar) until a thin lipid film is formed. The lipid film is thoroughly dried at 40 °C under vacuum of about 3 to 5 mbar for approximately 60 minutes. The dry lipid film is cooled in an ice bath and is rehydrated with a cold (4 °C) glucose or trehalose solution (pH 5-6) resulting in a suspension of multilamellar lipid vesicles at a total concentration of about 10 to 20 mM. Once a homogeneous dispersion is formed (after 15-20 min) the liposomal dispersion is extruded (filtration under pressure) at temperatures between 4 °C and 40 °C, between 1 and 5 times through polycarbonate membranes of appropriate size, typically between 100 and 400 nm. Preferably, the liposome preparation is performed at a temperature of 4 °C due to observation (HPLC-monitored) that formation of degradation product is significantly reduced compared with preparations at higher temperatures. The formed liposomal dispersion are characterized by measuring the concentration of each component (HPLC) and by determination of the zeta potential and the liposomal size using standardized procedures. Liposomes comprising DOTAP with or without DOPC and SuccTXL-DOTAP are formed with molar ratios as shown in Table 2 and Table 3:

**Table 2:**

| Liposomal formulations containing SuccTXL-DOTAP, DOTAP and DOPC | | | | |
|---|---|---|---|---|
| **Formulation** | **Liposomal Composition** | | **Liposomal Size (PI)** | **Zeta Potential [mV]** |
| | Theoretical | Measured | | |
| DOTAP/DOPC/SuccTXL | 50/47/3 | 50.6/46.1/3.2 | 184 nm (0.20) | 58 |
| DOTAP/DOPC/SuccTXL | 50/45/5 | 49.8/44.3/5.9 | 187 nm (0.16) | 54 |
| DOTAP/DOPC/SuccTXL | 50/43/7 | 50.8/43.1/6.1 | 170 nm (0.11) | 53 |
| DOTAP/DOPC/SuccTXL | 50/41/9 | 50.7/40.8/8.5 | 178 nm (0.19) | 53 |
| DOTAP/DOPC/SuccTXL | 50/39/11 | 49.7/39.0/11.3 | 175 nm (0.16) | 50 |
| DOTAP/DOPC/SuccTXL | 50/37/13 | 51.0/37.7/11.3 | 161 nm (0.10) | 52 |
| DOTAP/DOPC/SuccTXL | 50/35/15 | 51.7/35.6/12.7 | 171 nm (0.22) | 50 |
| DOTAP/DOPC/SuccTXL | 50/33/17 | 52.5/33.6/13.9 | 205 nm (0.50) | 50 |

**Table 3:**

| Liposomal formulations containing SuccTXL-DOTAP and DOTAP | | | | |
|---|---|---|---|---|
| **Formulation Formulation** | **Liposomal Composition** | | **Liposomal Size [nm] (PI)** | **Zeta Potential [mV]** |
| | Theoretical | Measured | | |
| DOTAP/SuccTXL | 89/11 | 89.7/10.3 | 185 (0.40) | 62 |
| DOTAP/SuccTXL | 85/15 | 86.9/13.1 | 162 (0.10) | 60 |

Employing the lipid film method and the DOTAP/DOPC system, liposomes with up to about 14 mol% (according to SuccTXL) can be formulated as measured by HPLC. The zeta potential values decrease with an increase of the SuccTXL-DOTAP content from about 3 up to 17 mol% (according to SuccTXL). However, this trend is not observed for formulations without DOPC. The liposomal size is not significantly affected by the liposomal composition.

Similar to the SuccTXL-DOTAP, formulations of 2'-succinyl docetaxel-DOTAP are prepared. The loading capacity is in the ranges which are found for SuccTXL-DOTAP. Liposomal size, Pl value and zeta potential of formulations of 2'-succinyl docetaxel-DOTAP do not substantially differ from SuccTXL-DOTAP formulations. The similarities between both formulations may be due to the taxane backbone that both compositions have in common.

### 2.2. Liposome Preparation via Ethanol Injection Method

Liposomal suspensions can be obtained by the solvent injection method where a solution of lipids and the inventive composition typically in ethanol ("ethanol injection method") is injected into the aqueous solution which contains glucose or trehalose at an isotonic concentration (pH 5-6). Preferably, the ethanol injection is performed at a temperature of 4 °C due to observation (HPLC-monitored) that formation of degradation product is significantly reduced compared with preparations at higher temperatures. The suitable volume of the lipid solution in ethanol is injected under vigorous stirring. Typically the concentration of the lipid is in the order of 400 mM. However, concentrations different from that value (200 mM, and lower) can be applied as well. The final ethanol content of the prepared liposomal formulation is usually in the range between 2.5 and 5 %. In general, the medium size of the resulting liposomes can be controlled to some extend by the lipid concentration in the ethanolic solution. As an alternative to ethanol, other suitable solvents or mixtures thereof can also be employed. Typically, these are alcohols, ethers, chloroform, hydrocarbons, etc. As well solvents in the supercritical state can be applied, such as hydrocarbons, carbon dioxide, perfluorinated compounds, etc. Subsequently to the described formulation procedure, extrusion, dialysis, a concentration step or freeze drying can be performed.

The liposomal suspensions as obtained by the methods as described above do not have necessarily the desired size distribution. Therefore, an extrusion through a membrane of defined pore size (100-400 nm) can be performed subsequently. In our experiments we have usually performed extrusion through membranes of 200 nm pores size (Osmonics Inc., Poretics, polycarbonate 0.2 µm). Table 4 summarizes formulations that have been prepared via ethanol injection method. HPLC analysis confirm the composition of the prepared formulations.

**Table 4:**

| Liposomal formulations prepared via ethanol injection | | | |
|---|---|---|---|
| **Formulation** | **Concentration of the Ethanolic Solution** | **Liposomal size (PI)** | |
| | | before extrusion | after extrusion |
| DOTAP/DOPC/SuccTXL (50:39:11) | 400 mM | 347 nm (0.4) | 214 nm/0.17 |
| DOTAP/SuccTXL (89:11) | 400 mM | 213 nm (0.23) | 185 nm/0.28 |
| DOTAP/DOPC/SuccTXL (50:39:11) | 100 mM | 48 nm (0.7) | (no extrusion) |
| DOTAP/SuccTXL (89:11) | 200 mM | 41 nm (0.6) | (no extrusion) |

### 3. Liposomal Formulations with Pegylated Lipids

Pegylated liposomes are of interest because they are characterized by a prolonged circulation time in the blood system due to less interaction with blood components such as e.g. macrophages.

Pegylated formulations containing SuccTXL-DOTAP are prepared employing the lipid film or the ethanol injection method as described above. Preferably, the liposome preparation is performed at a temperature of 4 øC due to observation (HPLC-monitored) that formation of degradation product is significantly reduced compared with preparations at higher temperatures. The pegylated lipid DOPE-PEG2000, where a PEG-chain with a molecular weight of 2000 Da is covalently linked to the primary amino group of DOPE, is admixed to the respective organic solution containing the inventive composition and cationic and/or neutral lipids and/ or anionic lipids. Usually, formulations with 5 mol% DOPE-PEG2000 are formulated. Examples of those are listed in Table 5.

**Table 5:**

| Formulations containing pegylated lipids | | | |
|---|---|---|---|
| DOTAP/DOPC/**DOPE -PEG**/SuccTXL | **Liposomal Size (PI)** | **Observation during Extrusion (200 nm)** | **Liposomal Composition determined by HPLC** |
| 50:34:5:11 | 232nm (0.17) | no problems | 53.8:29.9:5.0:11.3 |
| 50:30:5:15 | 232nm (0.17) | little problems only in | 50.7:28.0:4.9:16.4 |
| 50:28:5:17 | 245nm (0.47) (before Extrusion) | impossible impossible | 50.2:27.7:4.7:17.4 |

Liposomal size and PI values show a successful liposome preparation. As measured, the liposomal size is typically increased by 30-50 nm compared with the non-pegylated liposomes. Whereas formulation with 11 and 15 mol% SuccTXL can be extruded without problems, the 17 mol% formulation is not homogeneous and the extrusion step can not be performed due to blockage of the extrusion m membrane. In all cases HPLC analytic data support the overall successful preparation of first pegylated liposomes containing the inventive composition.

### 4. Physicochemical Stability of Liposomal SuccTXL-DOTAP

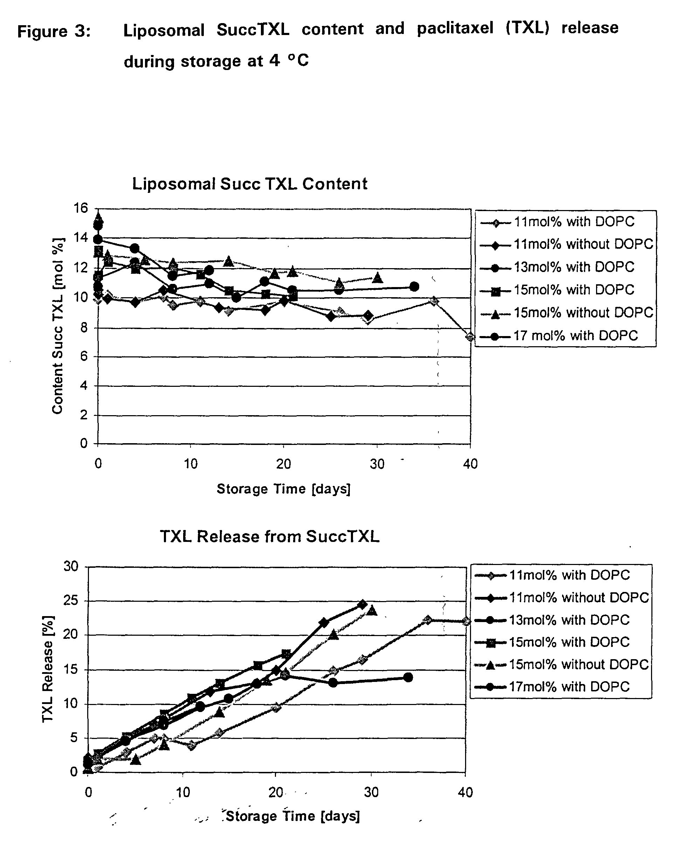

In order to investigate stability, SuccTXL-DOTAP containing liposomes (5% glucose) which are prepared following the lipid film method are stored for up to 30 days at 4 °C. At various time points the concentration of each component is determined (HPLC) and the formulations are characterized by liposomal size, zeta potential measurements and by determination of possible precipitation (microscopic evaluation). In addition, the chemical stability of succinyl paclitaxel is studied by determination of the released paclitaxel based on HPLC analysis. Long-time stability of various liposomal formulations monitored by the SuccTXL content (mol%) and the paclitaxel released from SuccTXL is shown in Figure 3.

The liposomal SuccTXL-DOTAP content in Fig. 3 refers to the total amount of lipid and is given as mol% of SuccTXL content. The paclitaxel release from the SuccTXL component of SuccTXL-DOTAP in Fig. 3 is calculated based on the initial amount of the SuccTXL component in the liposomes and is given as mol%. The results indicate very stable liposomal SuccTXL-DOTAP formulations with and without DOPC.

### 5. Lyophilization of Liposomal SuccTXL-DOTAP

For the lyophilization of formulations containing SuccTXL-DOTAP liposomes are prepared by the lipid film method or by ethanol injection (as described above) or another suitable method. Trehalose or another sugar or alcohol is used as cryoprotectant. Lyophilization of liposomes was performed according to a standard protocol.

Some examples of liposomal formulations that are lyophilized followed by reconstitution with the necessary volume of pure water are listed in Table 6.

**Table 6:**

| Characteristics of the lyophilized SuccTXL-DOTAP containing liposomes | | | | |
|---|---|---|---|---|
| **Formulation** | **Liposomal Composition** | | **Liposomal Size (PI)** | **Zeta Potential** |
| | Theoretical | Measured | | |
| DOTAP/DOPC/SuccTXL (10 mM total conc.) | 50/39/11 | 49.7/39.0/11.3 | 196 nm (0.03) | 55 mV |
| DOTAP/DOPC/SuccTXL (20 mM total conc.) | 50/39/11 | 49.7/40.5/9.8 | 200 nm (0.13) | 54 mV |
| DOTAP/SuccTXL (10 mM total conc.) | 89/11 | 90.5/9.5 | 179 nm (0.09) | 58 mV |
| DOTAP/SuccTXL (20 mM total conc.) | 89/11 | 88.2/11.8 | 209 nm (0.16) | 64 mV |

Liposomal formulations with and without DOPC are easily formed by reconstitution of the lyophilisates. The removed water volume was determined by weighing the vials before and after the lyophilization process. The average volume is taken of 5 different vials. Before use, the calculated volume is added to the lyophilisate, the vial is gently shaken and the homogenous solution is stored at 4 °C for 30 min to allow total degasification of the sample. The reconstituted liposomes were characterized as shown in Table 5. No significant paclitaxel release is observed during the lyophilization procedure as indicated by the HPLC data.

### 6. Physicochemical Stability of Lyophilized Liposomal SuccTXL-DOTAP

After reconstitution following the procedure as described above, the physicochemical stability of lyophilized liposomes is studied at 4 °C and at room temperature, respectively, over a period of 24 hours (so-called in-use stability). The compositions of the tested formulations are shown in Table 7:

**Table 7:**

| Lyophilized liposomal formulations | |
|---|---|
| **Formulation** | **Liposomal Composition** |
| DOTAP/DOPC/SuccTXL (10 mM total conc.) | 50/39/11 |
| DOTAP/DOPC/SuccTXL (20 mM total conc.) | 50/39/11 |
| DOTAP/SuccTXL (10 mM total conc.) | 89/11 |
| DOTAP/SuccTXL (20 mM total conc.) | 89/11 |

After reconstitution of the lyophilisates the reconstituted liposomes are kept at 4 °C or room temperature, respectively. At different time points (0 h, 4 h, 8 h and 24 h) the liposomes are characterized by size, zeta potential and determination of the concentration of each component by HPLC.

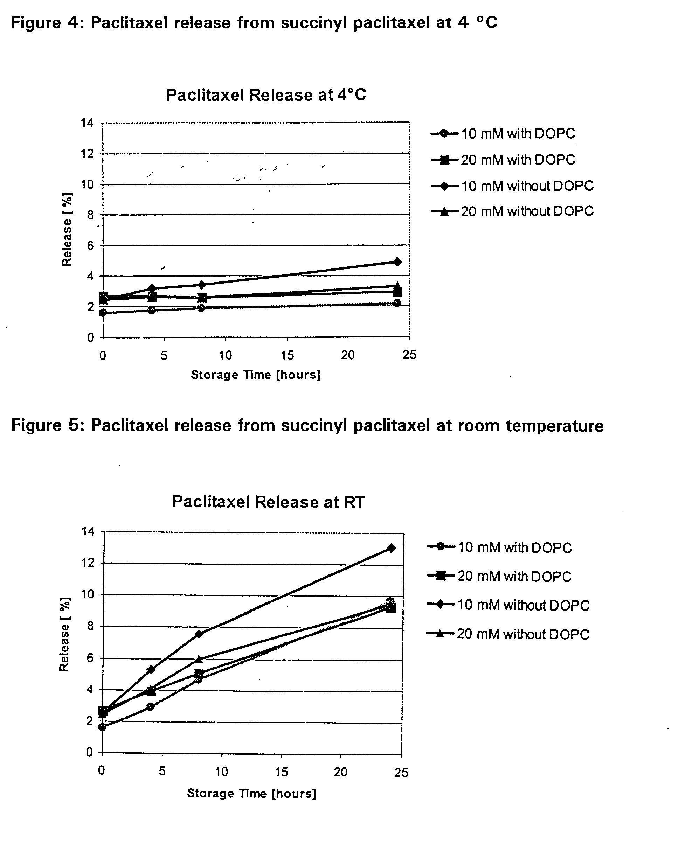

After 24 h storage at 4 °C or room temperature each formulation has liposomal diameters that are nearly identical to the liposomal size prior to lyophilization. In addition, no significant change of the zeta potential of each formulation is observed after the same treatment. HPLC analysis confirms excellent stability of the reconstituted lyophilisates. Whereas the concentrations of the lipid components do not change at all, none or only little paclitaxel release is found after 24 h storage: When stored at 4 °C none of the formulations reveals a significant instability of liposomal SuccTXL-DOTAP as monitored by measuring the amount of paclitaxel released from SuccTXL (SuccTXL-DOTAP) as shown in Figure 4.

Storage at room temperature results in the formation of 10-13 mol% paclitaxel with respect to the initial concentration of SuccTXL after 24 h (see Figure 5).

### 7. In Vitro Experiments

The efficacy of the liposomal succinyl paclitaxel formulation is determined in vitro by analysing the decrease of cell viability in correlation to the drug concentration. The drug concentration at which cell viability is inhibited to 50 % (lC₅₀) is used as index for the inhibitory potential.

A-375 (human melanoma cell line) and Ea.Hy 926 cells (transformed human endothelial cell line) are seeded at a constant density (2× 10⁴/cm²) in 24-well plates and cultivated over night at conditions of 5-5.5 % CO₂, 37 °C and ∼90% humidity. At day 1, cell culture medium is replaced by a mixture of fresh medium and a series of 11 consecutive drug dilutions is added to each well (duplicates) to cover a range between 0.1 and 1000 nM final drug concentration. After 72 h, the cell viability in each well is determined by measuring the activity of mitochondrial dehydrogenases (MTT assay). In viable cells the MTT substrate is converted to a blue, cell impermeable dye (Formazan). After 1 h the medium is removed, cells are lysed with isopropanol/0.04 % HCI and the amount of the blue Formazan given as optical density at a wavelength of 550 nm (OD₅₅₀ₙₘ) is quantitated in an ELISA reader. The experiment is evaluated using the Sigma Plot analysis software by plotting the mean OD₅₅₀ₙₘ value against the respective drug concentration. A best fit curve is calculated based on a double-sigmoid assumption algorithm and the lC₅₀ value is determined according to this best fit curve with results as shown in Table 8.

**Table 8:**

| Tested formulations and their IC₅₀ values | | |
|---|---|---|
| **Formulation** | **IC**_{**50**} **(A-375)** | **IC**_{**50**} **(Ea.Hy 926)** |
| Taxol® | 2 nM | 3 nM |
| SuccTXL | 10 nM | 17 nM |
| DOTAP/DOPC/SuccTXL (50/39/11) (LipoSpa 50) | 12 nM | 28 nM |

### 8. In Vivo Experiments

NMRI-nude mice were obtained from Elevage Janvier and housed in isolated ventilated cages under save environmental conditions (SPF facility, 22 °C, 30-70 % humidity, 12 h light/dark cycle) with food and water ad libitum. Experimental design was reviewed and approved by local authorities.

Mice were assigned to the experimental groups (8 animals per cage), housed and handled (including monitoring of the body weight gain) at least five days before tumor inoculation (= day -6 to 0). Tumor cells (A-375 human melanoma cell line, ATCC Nr.: CRL-1619) were grown as described in the data sheet supplied by ATCC. Tumor cells (5 × 10⁶ in PBS) were inoculated s.c. in the right dorsal flank of mice in a volume of 50 µl on day 0.

Drug treatment started after the tumors reached a volume of approximately 100 mm³. The drugs were given by iv injection, three times a week (Mo, Wed, Fri) for the following three weeks at equitoxic doses. The drugs were prepared as described. Equitoxic doses of the compounds had been determined in previous experiments, where toxicity was evaluated based on hematological parameters, body weight and clinical observations. The solutions were administered slowly in a volume of ∼10 µl/g body weight.

Animals were clinically monitored during the whole experiment and for at least one week after treatment was finished. Monitoring of tumor size was performed three times a week after start of treatment, before each application, during the treatment period and during the recovery period (at least one week). The tumor dimensions were measured by calliper and the tumor size was calculated according to the following formula: V = (LWý/6 (L = greatest length, W = width of perpendicular axis). The body weight of each individual animalswas monitored at least twice during handling period (e.g. day -6 and 0), after tumor inoculation, after start of treatment and during recovery period (at least one week) for all groups. EDTA blood was collected from the retrobulbar plexus at four different points: during handling (day -3), start of treatment (day 7), in the middle of treatment (∼day 21) and at the end of the recovery period (day 28) from 4 animals of all treatment groups for hematology. The number of red and white blood cells and platelets were determined using an automated cell counter (Abbott Cell Dyn 3500).

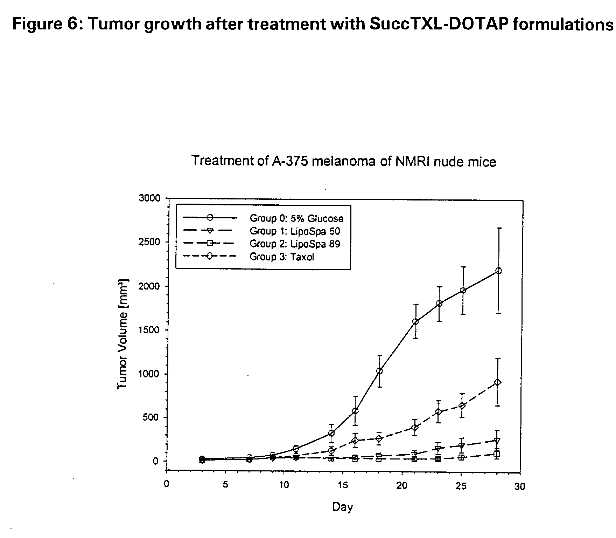

Experimental design and results of the animal experiments are shown in Figure 6 and Table 9. Whereas tumors in the control group showed a rapid and progressive tumor growth, all drug formulations reduced the tumor growth rate. Whereas both SuccTXL-DOTAP formulations showed a strong reduction in the tumor growth rate, Taxol® (reduced the tumor growth only to a limited extent.

**Table 9:**

| Tested formulations and doses | | | |
|---|---|---|---|
| **Group** | **Drug/Formulation** | **Dose [mg/kg]** | **Animals per Group** |
| 0 | 5 % Glucose | - | 8 |
| 1 | LipoSpa 50 DOTAP/DOPC/SuccTXL (50/39/11) | 12.5 | 8 |
| 2 | LipoSpa 89 DOTAP/SucctXL (89/11) | 10.0 | 8 |
| 3 | Taxol® | 5.0 | 8 |

### 1.9. Human Therapy Treatment Protocol

This example is concerned with human treatment protocols using the formulations disclosed. Treatment will be of use for preventing and/or treating various human diseases and disorders associated with enhanced angiogenic activity. It is considered to be particularly useful in anti-tumor therapy, for example, in treating patients with solid tumors and hematological malignancies or in therapy against a variety of inflammatory diseases such as psoriasis.

A feature of the invention is that several classes of diseases and/or abnormalities are treated without directly treating the tissue involved in the abnormality. Thus, by inhibiting angiogenesis the blood supply to a tumor e.g. is cut off and the tumor is killed without treating it directly.

Methods of treating such patients using lipid-drug complexes are known. It is contemplated that such methods may be straightforwardly adapted for use with the method described herein. As discussed above, other therapeutic agents could be administered either simultaneously or at distinct times. One may therefore employ either a pre-mixed pharmacological composition or "cocktail" of the therapeutic agents, or alternatively, employ distinct aliquots of the agents from separate containers.

The various elements of conducting a clinical trial, including patient treatment and monitoring, will be known to those of skill in the art in light of the present disclosure.

For regulatory approval purposes, it is contemplated that patients chosen for a study would have failed to respond to at least one course of conventional therapy and would have objectively measurable disease as determined by physical examination, laboratory techniques, or diagnostic imaging procedures. Such patients would also have no history of cardiac or renal disease and any chemotherapy should be stopped at least 2 weeks before entry into the study.

Prior to application the formulation can be reconstituted in an aqueous solution in case the formulation was freeze dried. Again, the required application volume is calculated from the patient's body weight and the dose schedule.

The disclosed formulations may be administered over a short infusion time. The infusion given at any dose level should be dependent upon the toxicity achieved after each. Hence, if Grade II toxicity was reached after any single infusion, or at a particular period of time for a steady rate infusion, further doses should be withheld or the steady rate infusion stopped unless toxicity improved. Increasing doses should be administered to groups of patients until approximately 60% of patients showed unacceptable Grade III or IV toxicity in any category. Doses that are 2/3 of this value would be defined as the safe dose.

Physical examination, tumor measurements, and laboratory tests should be performed before treatment and at intervals of about 3-4 weeks later. Laboratory tests should include complete blood counts, serum creatinine, creatine kinase, electrolytes, urea, nitrogen, SGOT, bilirubin, albumin, and total serum protein.

Clinical responses may be defined by acceptable measure or changes in laboratory values e.g. tumormarkers. For example, a complete response may be defined by the disappearance of all measurable disease for at least a month. Whereas a partial response may be defined by a 50% or greater reduction.

All of the compositions and methods disclosed and claimed herein can be made and executed without undue experimentation in light of the present disclosure. While the compositions and methods of this invention have been described in terms of preferred embodiments, it will be apparent to those of skill in the art that variations may be applied to the composition, methods and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA organization.

### Administration and dosing

The present invention includes a method of delivery of a pharmaceutically effective amount of the inventive formulation of a low molecular weight compound to an angiogenic vascular target site of a subject in need thereof. A "subject in need thereof" refers to a mammal, e.g. a human.

The route of administration comprises peritoneal, parenteral or topic administration and the formulations are easily administered in a variety of dosage forms such as injectable solutions, drug release capsules and the like.

For use with the present invention the term "pharmacologically effective amount" of a compound administered to a subject in need thereof (which may be any animal with a circulatory system with endothelial cells which undergo angiogenesis) will vary depending on a wide range of factors. The amount of the compound will depend upon the size, age, sex, weight, and condition of the patient as well as the potency of the substance being administered. Having indicated that there is considerable variability in terms of dosing, it is believed that those skilled in the art can, using the present disclosure, readily determine appropriate dosing by first administering extremely small amounts and incrementally increasing the dose until the desired results are obtained. Although the amount of the dose will vary greatly based on factors as described above, in general, the present invention makes it possible to administer substantially smaller amounts of any substance as compared with delivery systems which target the surrounding tissue e.g., target the tumor cells themselves.

The pharmaceutically effective amount of a therapeutic agent as disclosed herein depends on the kind and the type of action of the agent. For the examples mentioned herein, it is within the range of about 0.1 to about 20 mg/kg in humans. Typically, for paclitaxel derivatives doses in the order of about 5 mg/kg are applied.

## Claims

1. A composition comprising a derivative of a taxane drug with antimitotic activity which has a negative net charge (anionic derivative) and at least one amphiphile which has a positive net charge (cationic amphiphile), wherein said cationic amphiphile is a non-single chain amphiphile.

2. The composition of claim 1, wherein said taxane drug is selected from paclitaxel or docetaxel.

3. The composition of claim 1 or 2, wherein said anionic derivative is a taxane drug modified with a moiety having a negative net charge (negatively charged moiety).

4. The composition of any one of the claims 1 to 3, wherein said moiety is linked to the 7- or the 2'-hydroxyl group of the taxane drug, preferably to the 2'-hydroxyl group.

5. The composition of any one of the claims 1 to 4, wherein said moiety is linked via an ester or an ether bond, preferably an ester bond.

6. The composition of any one of the claims 1 to 5, wherein said moiety is selected from a molecule having at least one acidic group such as lactic acid, citric acid, succinic acid, glutaric acid, maleic acid, fumaric acid, malonic acid or adipic acid.

7. The composition of any one of the claims 1 to 6, wherein said composition is virtually free from the unmodified taxane drug.

8. The composition of any one of claims 1 to 7, wherein said cationic amphiphile is selected from lipids, lysolipids or pegylated lipids with a positive net charge, preferably a cationic amphiphile with a tertiary amino or a quaternary ammonium group such as N-[1-(2,3-diacyloxy)propyl]-N,N-dimethyl amine or N-[1-(2,3-diacyloxy)propyl]-N,N,N-trimethyl ammonium.

9. The composition of any one of the claims 1 to 8, further comprising at least one amphiphile which has a negative and/or neutral net charge (anionic and/or neutral amphiphile), preferably from sterols or lipids such as cholesterol, phospholipids, lysolipids, lysophospholipids, sphingolipids or pegylated lipids with a negative or neutral net change such as diacylphosphatidylcholine.

10. A colloidal nanoaggregate comprising a composition of any one of the claims 1 to 9, having an overall positive charge.

11. The nanoaggregate of claim 10 comprising an excess of positively charged moieties of at least 20 %, preferably at least 30 % and most preferably at least 40 % in the outer molecular layer.

12. The nanoaggregate of claim 10 or 11, which is present as an emulsion droplet, a liposome, a micelle or a nanoparticle.

13. The nanoaggregate of any one of the claims 10 to 12, comprising about 1 to 50 mol %, preferably 8 to 20 mol % of a derivative of a taxane drug.

14. The nanoaggregate of any one of the claims 10 to 13, which is a particle having an average particle size ranging from about 5 nm to 1 æm, preferably from about 25 nm to 500 nm and more preferably from about 100 nm to 300 nm.

15. The nanoaggregate of any one of the claims 10 to 14, further comprising a cryoprotectant selected from a sugar or an alcohol or a combination thereof such as trehalose, maltose, sucrose, glucose, lactose, dextran, mannitol or sorbitol.

16. A pharmaceutical preparation comprising a pharmaceutically effective amount of the composition of any one of the claims 1 to 9 or a colloidal nanoaggregate of any one of the claims 10 to 15 together with a pharmaceutically acceptable carrier, diluent and/or adjuvant.

17. A method of producing the colloidal nanoaggregate of any one of claims 10 to 15 comprising the steps of
a) providing a derivative of a taxane drug with antimitotic activity which has a negative net charge (anionic derivative) and
b) associating said derivative with a cationic amphiphile which has a positive net charge (cationic amphiphile)
c) and optionally at least one further amphiphile which has a positive, negative and/or neutral net charge (cationic, anionic and/or neutral amphiphile) forming a colloidal nanoaggregate.

18. The method of claim 17, wherein step b) and c) comprise forming said nanoaggregate by a homogenisation, a lipid film or by a solvent injection procedure.

19. The use of a pharmaceutical preparation of claim 16 for producing a medicament for treating and/or preventing a disease **characterized by** enhanced angiogenic activity.

20. The use of claim 19, wherein the taxane drug is provided at an angiogenic vascular target site by
a) liberating said derivative of a taxane drug from the associated cationic amphiphile and/or
b) cleaving off said negatively charged moiety, e.g. by hydrolysing a covalent bond.
